# EUROPEAN PATENT SPECIFICATION

(11) **EP 0 669 033 B1**
(45) Date of publication and mention of the grant of the patent: **03.09.1997**
(21) Application number: 93923874.7
(22) Date of filing: 12.10.1993
(51) Int. Cl.: G07F 11/44, B65D 5/42, A47F 1/03, B65D 5/72

(54) **EARPLUG DISPENSER BOX**
OHRPFROPFENAUSGABESCHACHTEL
BOITE DISTRIBUTRICE DE TAMPONS AURICULAIRES

(30) Priority: 15.10.1992 US 961432; 31.12.1992 US 999292
(43) Date of publication of application: 30.08.1995
(73) Proprietor: LEIGHT, Howard S., Santa Monica, CA 90404 (US)
(72) Inventor: LEIGHT, Howard S., Santa Monica, CA 90404 (US)
(74) Representative: Hackney, Nigel John
(86) International application number: US9309802
(87) International publication number: WO9409455

(56) References cited:
- GB-A- 1 287 082
- GB-A- 2 049 625
- US-A- 2 325 214
- US-A- 2 325 277
- US-A- 3 058 619
- US-A- 3 186 591
- US-A- 3 747 834
- US-A- 3 799 409
- US-A- 4 098 383
- US-A- 4 602 735
- US-A- 4 899 929

## Description

### BACKGROUND OF THE INVENTION:

Disposable earplugs, which each have a length of about 2.54cm (one inch) and a diameter of about 1.27cm (0.5 inch) can be conveniently packaged in cardboard boxes that each hold several hundred. The earplugs can be transferred from the box into a dispenser. It would be desirable if the contents of the box could be transferred without spillage, and without contact with a person's hands which could dirty the earplugs. Such box would be desirable for the transfer of other small articles.

United States patent no. US3799409 discloses a shippable container of corrugated board for handling and dispensing flowable material. The container includes a closure arrangement for its bottom comprising a pair of opposing flaps turned inwardly in their closed positions. A cover member is held on the container which when removed permits the opening of the discharge opening.

In a first aspect, the present invention provides a container which can hold multiple earplugs and which can be inserted into an open top portion of an earplug dispenser where the container can be readily opened to allow the earplugs to fall into the dispenser, where the container includes a box having side walls and a bottom wall, with a first of said walls having a lower edge, said bottom wall including at least a first flap having an inner end pivotally mounted on said lower edge of said first side wall and having an outer flap portion, characterised by:
a release device having a forward part coupled to said outer flap portion and to another part of said box to support said outer flap portion from moving down, said release device having a rear release part forming a handle which can be pulled rearwardly to decouple said forward part of said release device from said outer flap portion and allow said flap to pivot down.

In a second aspect, the present invention provides a method for holding and then releasing earplugs into a dispenser that has an open top, comprising:
forming a box with side walls and a bottom wall, including forming said bottom wall with a pair of flaps that have upwardly-facing and downwardly-facing flap surfaces, that each have inner ends pivotally mounted on opposite side walls and adjacent outer ends, including forming each flap outer end portion with a rearwardly-extending slot;
inserting a plate into said slots so a middle plate portion lies under said flap outer portions between said slots, and outer plate portions lie on top said flaps outward of said slots, to prevent said flaps from pivoting down until said plate is removed.

### SUMMARY OF THE INVENTION

In accordance with one embodiment of the present invention, a container is provided which can readily release multiple small articles such as earplugs held therein, into a receiver such as a dispenser, without involving handling by human hands and without substantial danger of spillage. The container includes a box that has a bottom wall with at least one flap. The flap has an inner end pivotally mounted at the bottom of a side wall of the box, and has a free outer end portion. A release device has a forward part coupled to the outer flap portion and to another portion of the box, to support the outer portion of the flap on another portion of the box, until the release device is pulled out to release the flap and allow it to pivot down under the weight of articles in the box.

The bottom wall of the box can include two flaps having inner ends pivotally connected to opposite side walls of the box, and having adjacent outer end portions. Each flap outer end portion has a slot forming a tab. The release device can be in the form of a plate whose inner portion lies in the slot to support the tabs, with widely spaced opposite sides of the plate supported on upper surfaces of the flaps.

### BRIEF DESCRIPTION OF THE DRAWINGS

Fig. 1 is an isometric and partially broken-away view of an earplug dispenser, showing a box of the present invention installed thereon.

Fig. 2 is an isometric view of the box of Fig. 1 in a closed configuration and also showing in phantom lines, the bottom flaps in an open configuration.

Fig. 3 is a view similar to that of Fig. 2, with the release device shown in solid lines in the release position and in phantom lines in the retain position.

Fig. 4 is a partial plan view of the release device and of a portion of the bottom wall flaps of Fig. 3.

Fig. 5 is a view taken on the line 5 - 5 of Fig. 4.

Fig. 6 is a partial isometric view of the apparatus of Fig. 4.

Fig. 7 is a front elevation view of the box of Fig. 3, shown prior to unfolding of the box.

Fig. 8 is a rear elevation view of a box in the configuration of Fig. 7.

Figs. 9 and 10 are partial isometric views of another container.

Fig. 11 is a partial isometric view of another container.

Fig. 12 is a partial isometric view of another container.

### DESCRIPTION OF THE PREFERRED EMBODIMENTS

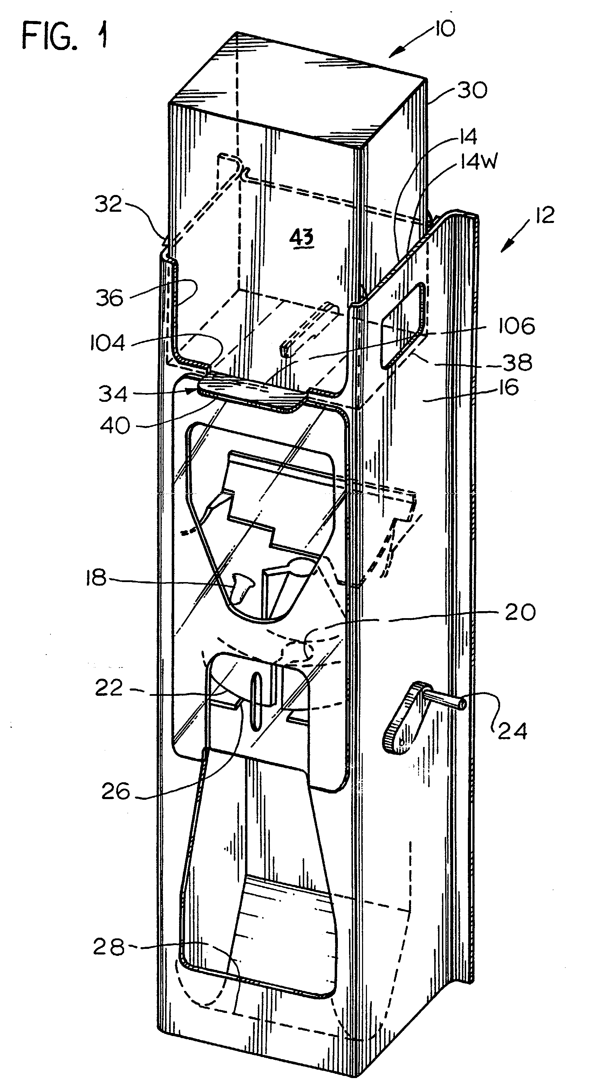

Fig. 1 illustrates a container 10 of the present invention, which is filled with earplugs and which is shown installed on an earplug dispenser 12. The earplug dispenser has an open top 14 for receiving the container, and has a frame 16 that forms a hopper for receiving the earplugs. One of the earplugs is indicated at 18. The earplugs fall into holes 20 of a wheel 22 that turns when a crank 24 is turned, to bring each hole and a corresponding earplug therein to a location over a dispense passage 26. The earplug drops out of the hole through the dispense passage, into a workman's hand or into a receiver 28 from which earplugs are removed. The container 10 and dispenser 12 are constructed so that a person can load the earplugs initially held in the container into the dispenser and can operate the dispenser to dispense a pair of earplugs, without having any worker's hands (which may be dirty) touch the earplugs.

The container 10 shown in Fig. 1 includes a box 30 which is installed on the dispenser by pressing the box down through the open top 14 of the dispenser, until stops 32 on the box lie a small distance above the walls 14w of the open top. At that time, a release device 34 of the container will lie within an opening 36 of the dispenser, with the bottom 38 of the box lying substantially at the bottom of the dispenser opening 36. An outer or rear end 40 of the release device forms a handle that can be pulled out, which allows a bottom wall of the box to open and the earplugs to drop out into the dispenser hopper.

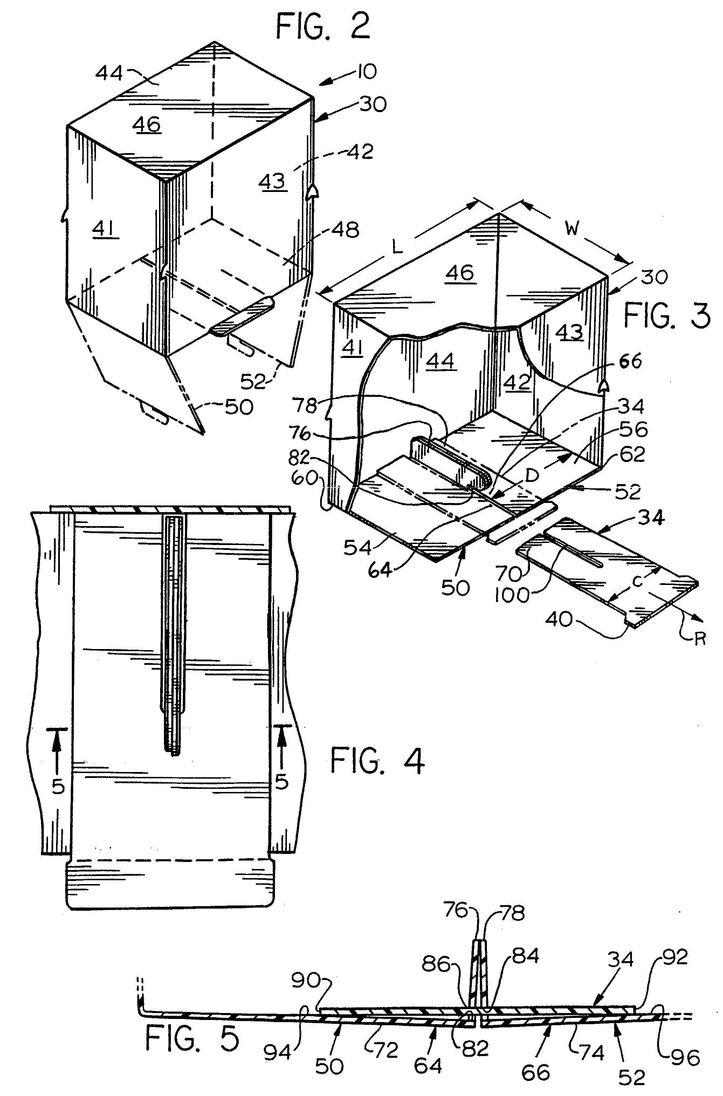

As shown in Fig. 2, the box has four side walls 41 - 44, a top wall 46, and a bottom wall 48. The bottom wall includes first and second flaps 50, 52. As shown in Fig. 3, each flap has an inner end 54, 56 pivotally mounted on the lower edge 60, 62 of corresponding opposite side walls 41, 42. Each flap also has an outer flap portion 64, 66. The release device 34 has a forward part 70 coupled to the outer flap portions 64, 66 to support each flap portion from moving down. When the release device handle 40 is pulled out in the rearward direction of arrow R, the flap outer portions are released to pivot down. The flaps pivot down under the weight of the articles contained in the box, and because of a tendency of the box to return to an unfolded configuration.

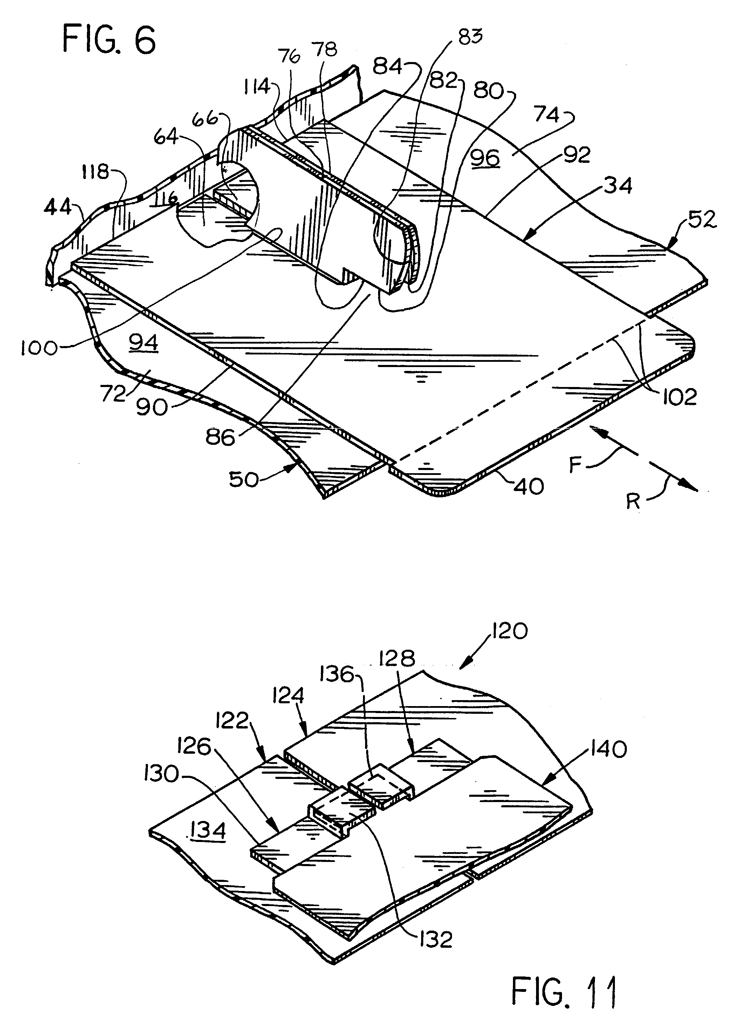

As shown in Fig. 6, each of the flaps has a main part 72, 74 both lying in a substantially horizontal plane. Each flap outer portion includes a largely vertical tab 76, 78 which is engaged with the release device 34. Each tab has a slot 80, 82 extending forwardly from a rear edge 83, with an upper slot wall forming a downwardly-facing surface or shoulder 84. The release device is preferably in the form of a rigid plate of material such as cardboard. The release device has a middle portion 86 with locations that bear against the downwardly-facing surfaces 84 of the tabs, and has opposite sides 90, 92 that bear against upwardly-facing surfaces 94, 96 of the flap main parts 72, 74.

As shown in Fig. 5, the middle portion 86 of the substantially rigid plate-like release device 34 presses up against the downwardly-facing surfaces 84 of the tabs 76, 78. At the same time, the opposite sides 90, 92 of the release device press down against the upwardly-facing surfaces 94, 96 of the flap main parts 72, 74. As the flaps 50, 52 begin to pivot downwardly, the tabs 76, 78 and the middle 86 of the release device, tend to move down much further than the opposite sides 90, 92 of the release device. As a result, the opposite sides 90, 92 of the release device are supported on the flaps, and the middle 86 of the release device supports the tabs 76, 78 and therefore the outer end portions of the flaps. Thus, the release device prevents the outer portions of the flaps from moving down by more than a small amount, to thereby prevent release of the articles in the box. When the release device 34 is pulled out, the flaps are free to move down and release the articles. Applicant prefers to construct the release device so its maximum width C (Fig. 3) where it engages the upper surfaces of the flaps, is at least 20 percent of the width D of each of the flaps, and preferably is at least 40 percent of the width of the flaps.

Referring again to Fig. 6, it can be seen that the release device 34 has a rearwardly-extending slot 100 which receives the two tabs 76, 78. This prevents more than small movement of the release device, except in rearward and forward directions R, F.

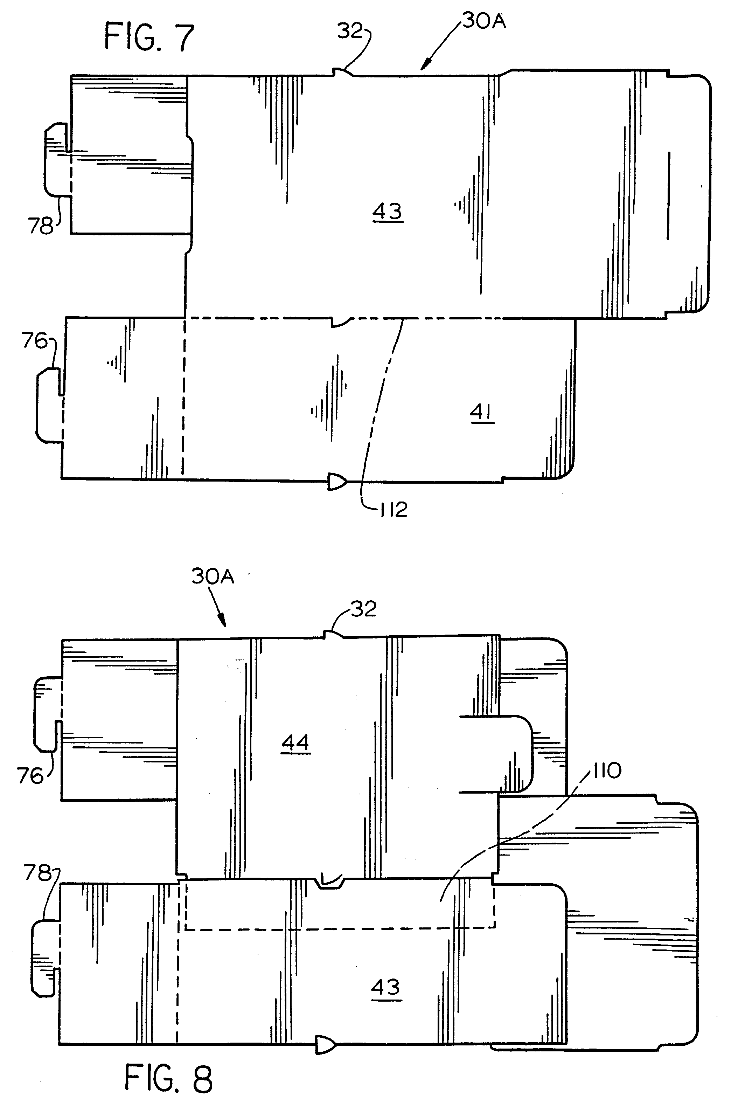

Figs. 7 and 8 illustrate the box at 30A in its manufactured but flat orientation. The box is formed from a single sheet of cardboard which has been bent as shown, and with the ends of the single sheet overlapping at 110 in Fig. 8. The stops 32 are formed by cuts along the corners such as at 112 where a pair of adjacent side walls meet. When the box is folded to its use position for fill up with earplugs, all of the stops 32 project and leave only small holes in the box. The release device 34 (Fig. 3) also can be formed of a sheet of cardboard.

Applicant prefers to construct the release device 34 (Fig. 6) so its front end 114 substantially abuts the rearwardly facing surface 116 of the rear box side wall 44, across the width of the release device. The release device thereby couples the bottom wall flaps 50, 52 to the rear wall 44. This enables the release device to help keep the bottom of the rear wall extending parallel to the inner edges 118 of the flaps, to avoid a gap through which earplugs could escape.

Applicant has constructed and successfully tested containers which were largely of the configuration shown in Figs. 1 - 8. Each box, designed to hold 200 earplugs, was of cardboard of 2.5mm thickness. Each flap (Fig. 3) had a width D of 5.6cm and length of 12.2cm. The release device had a width C of 7.6cm. The width W of the box was 11.2cm, the length L was 12.7cm, and the height was 18.5cm.

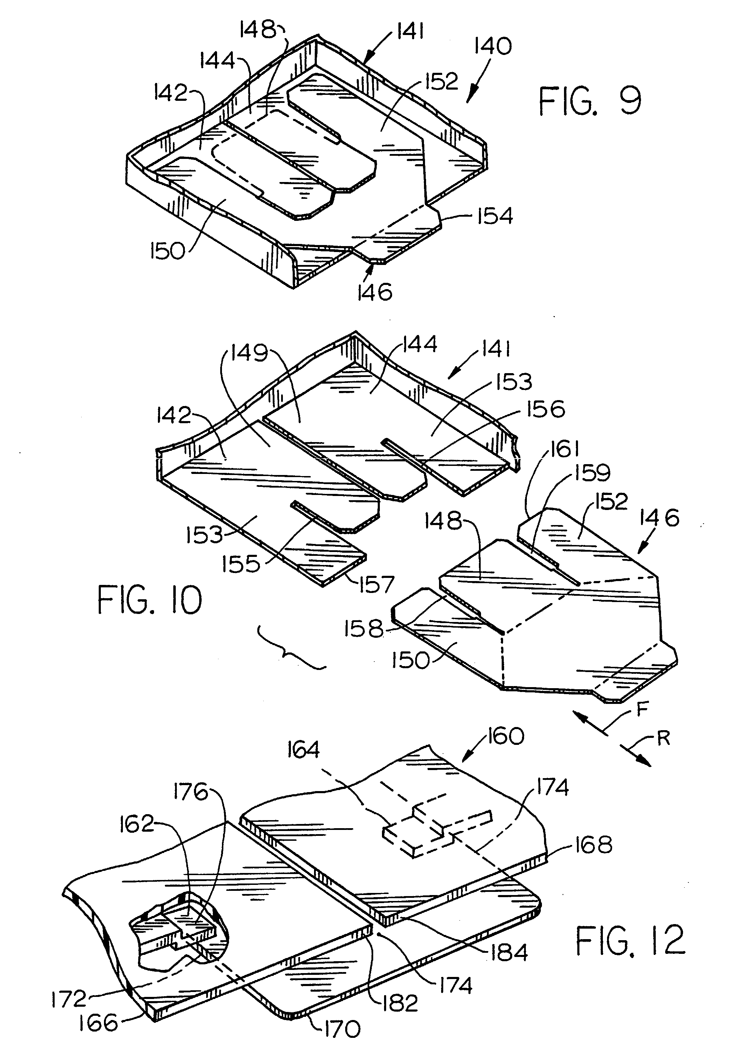

Fig. 9 and 10 illustrate a portion of another container 140 which includes a box 141 with a pair of flaps 142, 144 forming much of its bottom wall. A release device 146 has a middle 148 which lies under flap outer locations 149, and has opposite outer ends 150, 152 that lie over the inner flap locations 153. A handle 154 can be used to pull out the release device. Each of the flaps has a slot 155, 156 extending forwardly from a forward side 157 of each flap. The release device has a pair of slots 158, 159 extending from its forward edge 161. An important advantage of this container is that the flaps 142, 144 do not have upstanding tabs, on which one or two earplugs might remain when the flaps pivot down.

Fig. 11 illustrates a portion of another container 120 which includes a pair of flaps 122, 124 forming much of its bottom wall. However, instead of forming the flaps with upstanding tabs as extensions of the outer edges of the flaps, the tabs are in the form of separate rigid tabs 126, 128 having inner parts 130 fastened as by adhesive or staples to the corresponding flaps 122, 124. Each tab also has an upstanding portion 132 slightly spaced from an upper surface 134 of a corresponding flap to receive an inner portion 136 of a release device 140. The tab upstanding portion 132 may be considered as a location on the flap. The particular tabs 126, 128 can be formed of rigid plastic. The release device will act in the same manner as for the embodiment of Figs. 1 - 8 to prevent the flaps from pivoting down until the release device is pulled out.

Fig. 12 illustrates a portion of another container 160 which includes a pair of tabs 162, 164 lying below the bottom surfaces of the flaps 166, 168. A release device 170 in the form of a plate lies under the flaps. The release device has an inner end with opposite sides 172, 174 captured in the tabs and abutting vertically-facing shoulders 176 of the tabs. The middle 180 of the release device abuts the outer flap portions 182, 184. The upwardly-facing shoulders 176 may be considered to be locations on the upper surfaces of the flaps.

Although applicant prefers to use two separate flaps, it is possible to use a single flap whose outer end is held by a release device to the side walk. The release device lies substantially no lower than the bottoms (60, 62 in Fig. 3) of the box side walls, to avoid a gap under the box frontmost side wall 43 (Fig. 1) through which earplugs can fall out of the dispenser. That is, the release device should not extend below the side wall bottoms by more than 10 percent of the width or the length of the box. The release device handle 40 should project at least 5mm beyond the box frontmost side wall, to project forward of the dispenser front (or side) wall and to be grasped by fingers. The release device should be removable by sliding it horizontally.

Applicant has tried several techniques to enable a box to be installed in a dispenser and earplugs in the box then to be released. One approach was to use a thin plastic sheet covering the bottom of the box, and upstanding blades on the dispenser to cut three sides of the sheet as the box was installed. However, the sharp-pointed blades were dangerous. Another technique was to use a pair of flaps at the bottom of the box, and to use a piece of folded-over tape which could be pulled to peel the tape from outer portions of the flaps and allow them to pivot down. Applicant's present approach has been found to assure reliable release of the flaps and closure of the box in a low cost construction.

While terms such as "horizontal", "vertical", etc. are used herein to describe the invention, it should be understood that the box can be transported in any orientation with respect to gravity. Although applicant has developed the container to hold earplugs, it is possible for the container to be used to hold other articles of the same order of magnitude as the earplugs (which each have a length of about 2.5cm).

Thus, the invention provides a container which can hold multiple small articles, especially earplugs, and which can be inserted into an open top of a dispenser or other receiver, where the box can be readily released to allow the articles to fall out. The box has a bottom wall forming at least one flap, and a release device is provided which has an inner end which holds an outer portion of the flap to another portion of the box. When the release device is moved to a release position, as by pulling it, the flap is released to pivot down. The bottom wall preferably includes two flaps. The release device has a middle portion which lies under locations at outer ends of the two flaps, and has widely spaced opposite sides which bear against upper surface locations of the flaps.

## Claims

1. A container (10) which can hold multiple earplugs and which can be inserted into an open top portion (14) of an earplug dispenser (12) where the container can be readily opened to allow the earplugs to fall into the dispenser, where the container includes a box (30) having side walls (41-44) and a bottom wall (48), with a first (41) of said side walls having a lower edge, said bottom wall including at least a first flap (50) having an inner end (54) pivotably mounted on said lower edge of said first side wall and having an outer flap portion (69), characterised by:
a release device (34) having a forward part (70) coupled to said outer flap portion (64) and to another part of said box to support said outer flap portion from moving down, said release device having a rear release part (40) forming a handle which can be pulled rearwardly to decouple said forward part of said release device from said outer flap portion and allow said flap to pivot down.

2. The container described in claim 1 wherein:
a second (42) of said side walls lies opposite said first side wall and has a lower edge;
said bottom wall includes a second flap (52) having an inner end (56) pivotally mounted on said second side wall lower edge and having an outer second flap portion (66) forming said another part of said box;
said release device forward part being coupled to said outer flap portions to prevent said flap outer portions from separating and therefore prevent their downward pivoting, until said release device is pulled.

3. The container described in claim 2 wherein:
said release device has a middle (86) which lies under locations on said outer flap portions of said first and second flaps to support said outer flap portions, said release device including a pair of opposite ends (90, 92) that lie on top of inner flap locations on said first and second flaps that lie inward of said outer flap portions so said first and second flaps support said release device.

4. The container described in claim 2 wherein:
said flaps each have a main part (70, 72) lying in a substantially horizontal plane and said outer portions each have a largely vertical tab (76, 78), and each tab has a slot (80,82) with an upper slot wall forming a largely downwardly-facing surface;
said release device forward part lies under and against said tab downwardly-facing surfaces, and said release device is slidably received in said slots, so as said release device handle is pulled rearwardly said release device moves out of said slots.

5. The container described in claim 4 wherein:
said tabs form downwardly facing surfaces at the upper ends of said slots;
said release device is in the form of a rigid plate having a width that is at least 20 per cent of the width of each of said flaps, said plate having opposite sides that bear against upper surfaces of said flaps, and said plate having a middle that bears against said downwardly facing surfaces of said tabs.

6. The container described in claim 4 wherein:
said box side walls include a frontmost side wall lying opposite said release device handle and having a rearwardly facing surface, and said flaps have upper surfaces and have inner edges lying adjacent to said frontmost side wall;
said release device comprises a rigid plate having opposite sides bearing against said upper surfaces of said flaps and a middle engaged with said flap outer portions;
said plate having a forward end which substantially abuts said frontmost side wall to keep its rearwardly facing surface substantially parallel to said flap inner edges.

7. The container described in claim 1 wherein:
said box side walls have lower ends and said side walls include a frontmost side wall;
said release device lies substantially no lower than the lower ends of said box side walls, and said handle of said release device extends at least 5mm rearward of said frontmost side wall.

8. The container described in claim 1 including:
an earplug dispenser which has a frame with a box-receiving open upper part constructed with walls that closely receive said box, said walls including a first wall with an opening through which said handle projects to a position outside said frame, to enable grasping and pulling of said handle.

9. The container described in claim 1 wherein:
said side walls include a second side wall lying opposite said first side wall, said second side wall having a lower edge and said bottom wall including a second flap pivotally mounted on said lower edge of said second side wall, with said first and second flaps having adjacent outer end portions, and with said first and second flaps each having upwardly and downwardly facing surfaces;
said release device forward portion has a middle (86) that engages said downwardly facing surfaces of said flaps at said outer end portions thereof, said release device having opposite sides (90,92) that each engages one of said upper surfaces of said flaps at locations spaced from said flap outer end portions, said release device being horizontally slidable to remove said forward portion from said flaps to allow said flaps to pivot down.

10. A method for holding and then releasing earplugs into a dispenser that has an open top, comprising:
forming a box (30) with side walls (41-44) and a bottom wall (48), including forming said bottom wall with a pair of flaps (50, 52) that have upwardly-facing and downwardly-facing flap surfaces, that each have inner ends pivotally mounted on opposite side walls and adjacent outer ends, including forming each flap outer end portion with a rearwardly-extending slot;
inserting a plate into said slots so a middle plate portion (86) lies under said flap outer portions between said slots, and outer plate portions lie on top said flaps outward of said slots, to prevent said flaps from pivoting down until said plate is removed.

## Patentansprüche

1. Behälter (10), der mehrere Ohrpfropfen aufnehmen kann und der in einen offenen oberen Abschnitt (14) einer Ohrpfropfenausgabevorrichtung (12) eingesetzt werden kann, worin der Behälter leicht geöffnet werden kann, sodass die Ohrpfropfen in die Ausgabevorrichtung fallen können, worin der Behälter eine Schachtel (30) mit Seitenwänden (41-44) und einer Bodenwand (48) umfasst, wobei eine erste (41) der Seitenwände eine untere Kante besitzt, wobei die Bodenwand zumindest eine erste Klappe (50) aufweist, deren inneres Ende (54) schwenkbar an der unteren Kante der ersten Seitenwand montiert ist und die einen äußeren Klappenabschnitt (64) aufweist, gekennzeichnet durch:
eine Freigabevorrichtung (34) mit einem vorderen Teil (70), der mit dem äußeren Klappenabschnitt (64) und einem anderen Teil der Schachtel verbunden ist, um den äußeren Klappenabschnitt zu stützen und daran zu hindern, sich hinunterzubewegen, wobei die Freigabevorrichtung einen hinteren Freigabeteil (40) besitzt, der einen Griff bildet, der nach hinten gezogen werden kann, um den vorderen Teil der Freigabevorrichtung vom äußeren Klappenabschnitt zu lösen und das Hinunterschwenken der Klappe zu ermöglichen.

2. Behälter nach Anspruch 1, worin:
eine zweite (42) Seitenwand gegenüber der ersten Seitenwand positioniert ist und eine untere Kante besitzt;
die Bodenwand eine zweite Klappe (52) mit einem inneren Ende (56) aufweist, die schwenkbar an der unteren Kante der Seitenwand montiert ist und einen äußeren zweiten Klappenabschnitt (66) aufweist, der den anderen Teil der Schachtel bildet;
der vordere Teil der Freigabevorrichtung mit den äußeren Klappenabschnitten verbunden ist, um eine Trennung der äußeren Klappenabschnitte und somit ihr Hinunterschwenken zu verhindern, bis die Freigabevorrichtung gezogen wird.

3. Behälter nach Anspruch 2, worin:
die Freigabevorrichtung einen Mittelabschnitt (86) aufweist, der unterhalb von Stellen auf den äußeren Klappenabschnitten der ersten und zweiten Klappe liegt, um die äußeren Klappenabschnitte zu stützen, wobei die Freigabevorrichtung ein Paar gegenüberliegender Enden (90, 92) umfasst, die oberhalb von inneren Stellen auf der ersten und zweiten Klappe liegen, die bezüglich der äußeren Klappenabschnitte innen liegen, sodass die erste und die zweite Klappe die Freigabevorrichtung stützen.

4. Behälter nach Anspruch 2, worin:
die Klappen jeweils einen Hauptteil (70, 72) aufweisen, der in einer im wesentlichen horizontalen Ebene liegt, und die Außenabschnitte jeweils eine größtenteils vertikale Lasche (76, 78) aufweisen, und jede Lasche einen Schlitz (80, 82) mit einer oberen Schlitzwand aufweist, die eine größtenteils nach unten gerichtete Oberfläche bildet;
der vordere Teil der Freigabevorrichtung unter den nach unten gerichteten Laschenoberflächen liegt und an diesen anliegt und die Freigabevorrichtung gleitend in den Schlitzen aufgenommen ist, sodass sich beim Ziehen des Griffs der Freigabevorrichtung nach hinten die Freigabevorrichtung aus den Schlitzen bewegt.

5. Behälter nach Anspruch 4, worin:
die Laschen nach unten gerichtete Oberflächen an den oberen Enden der Schlitze bilden;
die Freigabevorrichtung die Form einer starren Platte aufweist, deren Breite zumindest 20% der Breite jeder Klappe ausmacht, wobei die Platte gegenüberliegende Seiten besitzt, die an den oberen Flächen der Klappen anliegen, und die Platte einen Mittelabschnitt aufweist, der an den nach unten gerichteten Oberflächen der Laschen anliegt.

6. Behälter nach Anspruch 4, worin:
die Seitenwände der Schachtel eine vorderste Seitenwand enthalten, die gegenüber dem Griff der Freigabevorrichtung liegt und eine nach hinten gerichtete Oberfläche besitzt, und die Klappen obere Flächen und innere Kanten aufweisen, die angrenzend zur vordersten Seitenwand positioniert sind;
wobei die Freigabevorrichtung eine starre Platte mit gegenüberliegenden Seiten, die an den oberen Flächen der Klappen anliegen, und einen mit den äußeren Klappenabschnitten in Verbindung stehenden Mittelabschnitt umfasst;
wobei die Platte ein vorderes Ende aufweist, das im wesentlichen an der vordersten Seitenwand anliegt, um ihre nach hinten gerichtete Oberfläche im wesentlichen parallel zu den inneren Klappenkanten zu halten.

7. Behälter nach Anspruch 1, worin:
die Seitenwände der Schachtel untere Enden besitzen und die Seitenwände eine vorderste Seitenwand umfassen;
die Freigabevorrichtung im wesentlichen nicht niedriger als die unteren Enden der Seitenwände der Schachtel liegt und sich der Griff der Freigabevorrichtung von der vordersten Seitenwand zumindest 5 mm nach hinten erstreckt.

8. Behälter nach Anspruch 1, umfassend:
eine Ohrpfropfenausgabevorrichtung, die einen Rahmen mit einem schachtelaufnehmenden offenen Oberteil besitzt, der mit Wänden konstruiert ist, die die Schachtel satt aufnehmen, wobei die Wände eine erste Wand mit einer Öffnung umfassen, durch die der Griff zu einer Position außerhalb des Rahmens ragt, um das Fassen und Ziehen des Griffs zu ermöglichen.

9. Behälter nach Anspruch 1, worin:
die Seitenwände eine zweite Seitenwand enthalten, die gegenüber der ersten Seitenwand liegt, wobei die zweite Seitenwand eine untere Kante aufweist und die Bodenwand eine zweite Klappe enthält, die schwenkbar an der unteren Kante der zweiten Seitenwand montiert ist, wobei die erste und die zweite Klappe angrenzende äußere Endabschnitte besitzen und jeweils nach oben und unten gerichtete Oberflächen aufweisen;
der vordere Abschnitt der Freigabevorrichtung einen Mittelabschnitt (86) besitzt, der an den nach unten gerichteten Oberflächen der Klappen in ihren äußeren Endabschnitten angreift, wobei die Freigabevorrichtung gegenüberliegende Seiten (90, 92) aufweist, die jeweils an eine der oberen Flächen der Klappen an Stellen angreifen, die von den äußeren Klappenendabschnitten beabstandet sind, wobei die Freigabevorrichtung horizontal gleiten kann, um den vorderen Abschnitt von den Klappen zu entfernen, sodass die Klappen abwärtsschwenken können.

10. Verfahren zum Halten und anschließenden Abgeben von Ohrpfropfen in eine Ausgabevorrichtung, die einen offenen Oberteil aufweist, umfassend:
das Ausbilden einer Schachtel (30) mit Seitenwänden (41-44) und einer Bodenwand (48), einschließlich des Ausbildens der Bodenwand mit einem Klappenpaar (50, 52), das nach oben und nach unten gerichtete Klappenoberflächen aufweist, die jeweils innere Enden besitzen, die schwenkbar an gegenüberliegenden Seitenwänden und benachbarten äußeren Enden montiert sind, einschließlich des Ausbildens jedes äußeren Klappenendabschnitts mit einem sich nach hinten erstreckenden Schlitz;
Einsetzen einer Platte in die Schlitze, sodass ein mittlerer Plattenabschnitt (86) unter den äußeren Klappenabschnitten zwischen den Schlitzen liegt und die äußeren Plattenabschnitte über den Klappen außerhalb der Schlitze liegen, sodass die Klappen am Hinunterschwenken gehindert werden, bis die Platte entfernt wird.

## Revendications

1. Conteneur (10) qui peut retenir de multiples bouchons d'oreilles et qui peut être inséré dans une portion supérieure ouverte (14) d'un distributeur de bouchons d'oreille (12) dans lequel le conteneur peut être facilement ouvert pour permettre aux bouchons d'oreilles de tomber dans le distributeur, dans lequel le conteneur comprend une boîte (30) ayant des parois latérales (41-44) et une paroi de fond (48), avec une première (41) desdites parois latérales ayant un bord inférieur, ladite paroi de fond comprenant au moins un premier volet (50) ayant une extrémité interne (54) montée pivotante sur ledit bord inférieur de ladite première paroi latérale et ayant une portion externe de volet (69), caractérisé par :
un dispositif de libération (34) ayant une partie avant (70) accouplée à ladite portion externe de volet (64) et à une autre partie de ladite boîte pour supporter ladite portion externe de volet et l'empêcher de se déplacer vers le bas, ledit dispositif de libération ayant une partie de libération arrière (40) formant une poignée qui peut être tirée vers l'arrière pour désaccoupler ladite partie avant dudit dispositif de libération de ladite portion externe de volet et permettre audit volet de pivoter vers le bas.

2. Conteneur selon la revendication 1, dans lequel :
une seconde (42) desdites parois latérales repose à l'opposé de ladite première paroi latérale et présente un bord inférieur;
ladite paroi de fond comprend un second volet (52) ayant une extrémité interne (56) montée pivotante sur ledit bord inférieur de la seconde paroi latérale et ayant une seconde portion externe de volet (66) formant ladite autre partie de ladite boîte;
ladite partie avant du dispositif de libération étant accouplée aux dites portions externes de volet pour empêcher lesdites portions externes de volet de se séparer et par conséquent pour éviter leur pivotement vers le bas, jusqu'à ce que ledit dispositif de libération soit tiré.

3. Conteneur selon la revendication 2, dans lequel :
ledit dispositif de libération présente un milieu (86) qui repose sous des emplacements sur lesdites portions externes de volet desdits premier et second volets pour supporter lesdites portions externes de volet, ledit dispositif de libération comprenant une paire d'extrémités opposées (90, 92) qui reposent au-dessus d'emplacements internes de volet sur lesdits premier et second volets qui reposent vers l'intérieur desdites portions externes de volet de façon que lesdits premier et second volets supportent ledit dispositif de libération.

4. Conteneur selon la revendication 2, dans lequel :
lesdits volets comportent chacun une partie principale (70, 72) reposant dans un plan sensiblement horizontal et lesdites portions externes présentent chacune une languette en grande partie verticale (76, 78) et chaque languette présente une fente (80, 82) avec une paroi de fente supérieure formant une surface orientée en grande partie vers le bas;
ladite partie avant du dispositif de libération repose sous et contre lesdites surfaces orientées vers le bas des languettes, et ledit dispositif de libération est reçu coulissant dans lesdites fentes, de façon que lorsque ladite poignée du dispositif de libération est tirée vers l'arrière, ledit dispositif de libération se déplace hors desdites fentes.

5. Conteneur selon la revendication 4, dans lequel :
lesdites languettes forment des surfaces orientées vers le bas au niveau des extrémités supérieures desdites fentes;
ledit dispositif de libération est sous la forme d'une plaque rigide ayant une largeur qui est au moins 20 pour cent de la largeur de chacun desdits volets, ladite plaque ayant des côtés opposés qui appuient contre les surfaces supérieures desdits volets, et ladite plaque ayant un milieu qui appuie contre lesdites surfaces orientées vers le bas desdites languettes.

6. Conteneur selon la revendication 4, dans lequel :
lesdites parois latérales de la boîte comprennent une paroi latérale frontale extrême reposant à l'opposé de ladite poignée du dispositif de libération, et ayant une surface orientée vers l'arrière, et lesdits volets ont des surfaces supérieures et ont des bords internes reposant adjacents à ladite paroi latérale frontale extrême;
ledit dispositif de libération comprend une plaque rigide ayant des côtés opposés appuyant contre lesdites surfaces supérieures desdits volets et un milieu en contact avec lesdites portions externes de volet;
ladite plaque ayant une extrémité avant qui vient substantiellement buter contre ladite paroi latérale frontale extrême pour maintenir sa surface orientée vers l'arrière sensiblement parallèle auxdits bords internes de volets.

7. Conteneur selon la revendication 1, dans lequel :
lesdites parois latérales de la boîte ont des extrémités inférieures et lesdites parois latérales comprennent une paroi latérale frontale extrême;
ledit dispositif de libération repose sensiblement pas plus bas que les extrémités inférieures desdites parois latérales de la boîte, et ladite poignée dudit dispositif de libération s'étend au moins 5mm vers l'arrière de ladite paroi latérale frontale extrême.

8. Conteneur selon la revendication 1, comprenant :
un distributeur de bouchons d'oreille qui comprend un châssis avec une partie supérieure ouverte réceptrice de boîte construite avec des parois qui reçoivent étroitement ladite boîte, lesdites parois comprenant une première paroi avec une ouverture à travers laquelle ladite poignée fait saillie vers une position à l'extérieur dudit châssis, pour permettre la préhension et la traction de ladite poignée.

9. Conteneur selon la revendication 1, dans lequel :
lesdites parois latérales comprennent une seconde paroi latérale reposant à l'opposé de ladite première paroi latérale, ladite seconde paroi latérale ayant un bord inférieur et ladite paroi inférieure comprenant un second volet monté pivotant sur ledit bord inférieur de ladite seconde paroi latérale, avec lesdits premier et second volets ayant des portions d'extrémité externes adjacentes, et avec lesdits premier et second volets ayant chacun des surfaces orientées vers le haut et vers le bas;
ladite portion avant du dispositif de libération comporte un milieu (86) qui vient en contact avec lesdites surfaces orientées vers le bas desdits volets au niveau desdites portions d'extrémité externes de ces derniers, ledit dispositif de libération ayant des côtés opposés (90, 92) qui viennent en contact chacun avec l'une desdites surfaces supérieures desdits volets en des emplacements espacés desdites portions d'extrémité externes de volet, ledit dispositif de libération étant horizontalement coulissant pour retirer desdits volets ladite portion avant pour permettre auxdits volets de pivoter vers le bas.

10. Procédé pour retenir et puis relâcher des bouchons d'oreille dans un distributeur qui présente un sommet ouvert, consistant :
à former une boîte (30) avec des parois latérales (41-44) et une paroi inférieure (48), comprenant le formage de ladite paroi inférieure avec une paire de volets (50, 52) qui ont des surfaces de volet orientées vers le haut et vers le bas, qui ont chacune des extrémités internes montées pivotantes sur des parois latérales opposées et des extrémités externes adjacentes, comprenant le formage de chaque portion d'extrémité externe de volet avec une fente s'étendant vers l'arrière;
à insérer une plaque dans lesdites fentes de façon qu'une portion de plaque médiane (86) repose sous lesdites portions externes de volet entre lesdites fentes, et des portions externes de plaque reposent au-dessus desdits volets à l'extérieur desdites fentes, pour empêcher lesdits volets de pivoter vers le bas jusqu'à ce que ladite plaque soit retirée.
